# EUROPEAN PATENT SPECIFICATION

(11) **EP 4 460 495 B1**
(45) Date of publication and mention of the grant of the patent: **08.10.2025**
(21) Application number: 22843195.3
(22) Date of filing: 20.12.2022
(51) Int. Cl.: C07D 251/24

(54) **PROCESS FOR ISOLATING 2,4-BIS-(2,4-DIHYDROXYPHENYL)-6-(4-METHOXYPHENYL)-1,3,5-TRIAZINE (DIOPAT)**
VERFAHREN ZUR ISOLIERUNG VON 2,4-BIS-(2,4-DIHYDROXYPHENYL)-6-PHENYL-1,3,5-TRIAZIN (DIOPAT)
PROCÉDÉ POUR ISOLER 2,4-BIS-(2,4-DIHYDROXYPHÉNYL)-6-(4-MÉTHOXYPHÉNYL)-1,3,5-TRIAZINE (DIOPAT)

(30) Priority: 07.01.2022 EP 22150571
(43) Date of publication of application: 13.11.2024
(73) Proprietor: BASF SE, 67056 Ludwigshafen am Rhein (DE)
(72) Inventor: SCHWEDE, Christian, 69469 Weinheim (DE); HAUS, Johannes Felix, 67056 Ludwigshafen am Rhein (DE); THERRE, Joerg, 67056 Ludwigshafen am Rhein (DE); MALISZ, Jacek, 67056 Ludwigshafen am Rhein (DE); KRONEMAYER, Helmut, 67056 Ludwigshafen am Rhein (DE); KRUCK, Matthias, 4133 Schweizerhalle (Muttenz) (CH); SPITTLER, Markus, 79639 Grenzach-Wyhlen (DE)
(74) Representative: BASF IP Association
(86) International application number: PCT/EP2022/087021
(87) International publication number: WO 2023/131521

(56) References cited:
- EP-A1- 0 775 698
- WO-A1-2016/184764
- WO-A1-2020/016366
- WO-A1-2020/089323

## Description

The present invention provides an improved process for isolating 2,4-bis-(2,4-dihydroxyphenyl)-6-(4-methoxyphenyl)-1,3,5-triazine (DIOPAT) from a DIOPAT-containing aqueous mixture N, wherein the process comprises the steps of concentrating the aqueous mixture N to obtain a DIOPAT-containing aqueous suspension S1 having a certain solid content; evaporating solvent from the aqueous suspension S1; and drying the DIOPAT to obtain a solid content of more than 90 wt.-%, based on the total weight of the DIOPAT as well as DIOPAT obtained by the improved process having a particular bulk density.

2,4-bis-(2,4-dihydroxyphenyl)-6-(4-methoxyphenyl)-1,3,5-triazine (DIOPAT) is the starting material for the preparation of the UV absorber Tinosorb^{®} S (also known as 2,2-[6-(4-methoxyphenyl)-1,3,5-triazine-2,4-diyl] bis{5-[(2-ethylhexyl)oxy]phenol}, anisotriazine, bis-ethylhexyloxyphenol methoxyphenyl triazine, or bemotrizinol; CAS Number 187393-00-6) having the following chemical formula.

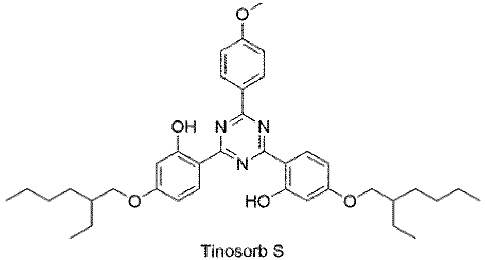

Tinosorb^{®} S is a broad band UV absorber, absorbing UVB as well as UVA rays. Thus, Tinosorb^{®} S is an important ingredient for sunscreen compositions and cosmetic applications.

One possible synthesis route to DIOPAT is performed via two steps, starting from 4-bromoanisole and cyanuric chloride under Grignard conditions to form the intermediate DICAT. In the second synthesis step, DICAT is reacted with resorcinol in a Friedel-Crafts reaction to form DIOPAT. In the following, the synthesis route to DIOPAT, starting from 4-bromoanisole and cyanuric chloride, is depicted, wherein the parameters a) Mg, THF; b) cyanuric chloride, THF; and c) resorcinol, toluene/benzonitrile, AlCl₃ are typically applied.

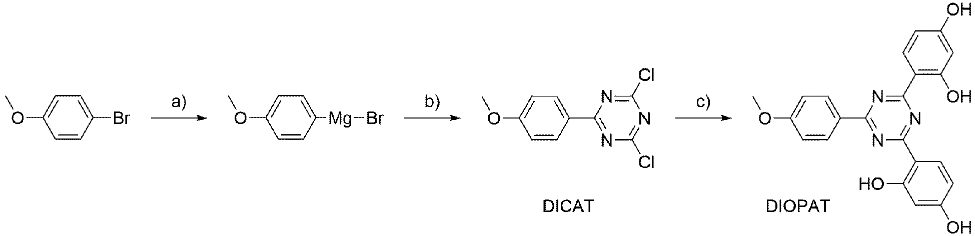

To complete the syntheses to Tinosorb^{®} S, a third step, the alkylation of DIOPAT with isooctyl chloride, is typically performed. In the following, the reaction to Tinosorb^{®} S is depicted, wherein the parameters a) isooctyl chloride, base, DMF are typically applied.

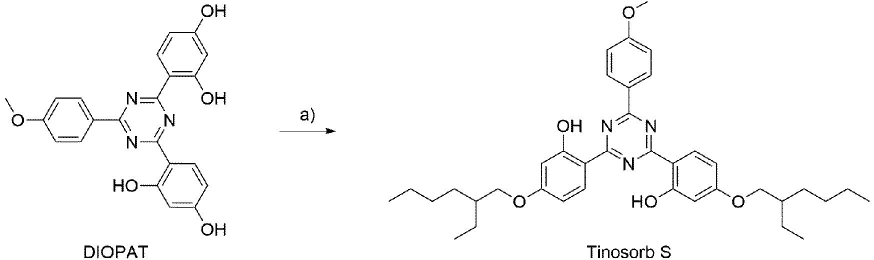

In connection with the preparation of DIOPAT, the workup procedure and isolation of DIOPAT causes difficulties.

Typically, the reaction mixture comprising DIOPAT is quenched on a pre-charged sodium hydroxide solution. The product DIOPAT as well as the aluminum salts (Al-salts) from the Friedel-Crafts reaction are then dissolved in the alkaline aqueous sodium hydroxide solution. The organic reaction solvent (e.g. a mixture of toluene and benzonitrile) is separated by phase separation. Residual organic solvents may be stripped off to guarantee an organic solvent free aqueous phase. Then, the DIOPAT is precipitated from the alkaline DIOPAT/Al-salt solution by acidifying the mixture. If a low pH is established (pH < 1), the Al-salts are still dissolved in the aqueous phase while the DIOPAT is precipitated as a solid.

However, standard filtration processes, such as the use of a filter press, to separate the precipitated DIOPAT from the Al-salt solution have disadvantages. In particular, the filtration process is a manual, time consuming and open process, which is economically unattractive and causes safety issues on technical scale. Furthermore, the DIOPAT will be obtained together with the undesired organic impurity 2,4-dihydroxybenzophenone (2,4-DHBP), which is a byproduct of the DIOPAT preparation. Impurities of the undesired by-product of 2,4-DHBP in the DIOPAT, which is used for the final reaction step to Tinosorb^{®} S, elevates the consumption of the expensive reactant isooctyl chloride and results in undesired side products, thus increasing the production costs.

An improved separation of the Al-salts from DIOPAT is challenging. Due to the low solubility of DIOPAT in organic solvents that have a complete miscibility gap between the aqueous and the organic phase, a separation of the Al-salts from DIOPAT by phase separation is not suitable. On the other hand, due to the corrosive behavior of acidic AlCl₃/DIOPAT suspensions, most filtration equipment where metallic material is in contact with these suspensions is not suitable. However, the Al-salts, as well as organic by-products, obtained in the Friedel-Crafts reaction with AlCl₃ are unfavorable for the following reaction to the final Tinosorb^{®} S and need to be separated from DIOPAT.

WO2020089323 and WO202016366 provide a separation technology based on ultrafiltration. The obtained suspension may be dried via spray drying, which is however energy consuming.

Therefore, it was an object of the present invention to provide an energy efficient process of isolating DIOPAT.

Further, it was the object of the present invention to provide an improved process for isolating DIOPAT from the DIOPAT/Al-salt solution obtained after quenching the reaction mixture of the Friedel-Crafts reaction for preparing DIOPAT and removing the organic solvents.

It is a further object of the present invention to provide a process for isolating DIOPAT, which avoids a manual, time consuming and open filtration process.

It is another object of the present invention to provide an improved process for isolating DIOPAT, wherein not only aluminum salts but also organic by-products are simultaneously separated from the DIOPAT.

It is another object of the present invention to provide an improved process for isolating DIOPAT, wherein the bulk density of DIOPAT is improved.

It has surprisingly been found that at least one of these objects can be achieved by a process for isolating 2,4-bis-(2,4-dihydroxyphenyl)-6-(4-methoxyphenyl)-1,3,5-triazine (DIOPAT) from a 2,4-bis-(2,4-dihydroxyphenyl)-6-(4-methoxyphenyl)-1,3,5-triazine-containing aqueous mixture N comprising the steps of
a) concentrating the aqueous mixture N to obtain a 2,4-bis-(2,4-dihydroxyphenyl)-6-(4-methoxyphenyl)-1,3,5-triazine-containing aqueous suspension S1 having a solid content of 3 to 30 wt.-%, based on the total weight of the aqueous suspension S1;
b) evaporating solvent from the aqueous suspension S1 by means of an agitated contact dryer; and
c) drying the 2,4-bis-(2,4-dihydroxyphenyl)-6-(4-methoxyphenyl)-1,3,5-triazine to obtain a solid content of more than 90 wt.-%, based on the total weight of the 2,4-bis-(2,4-dihydroxyphenyl)-6-(4-methoxyphenyl)-1,3,5-triazine.

Preferred embodiments of the present invention can be found in the claims, the description and the examples. It is to be understood that the features mentioned above and those still to be illustrated below of the subject matter of the invention are preferred not only in the respective given combination but also in other combinations without leaving the scope of the invention.

Before describing in detail exemplary embodiments of the present invention, definitions important for understanding the present invention are given.

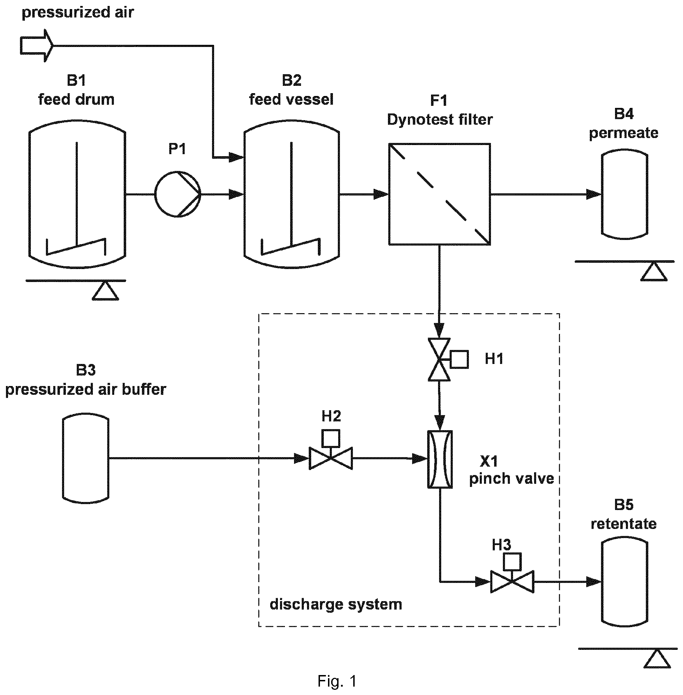

Figure 1 denotes a schematic microfiltration.

As used in this specification and in the appended claims, the singular forms of "a" and "an" also include the respective plurals unless the context clearly dictates otherwise. In the context of the present invention, the term "about" or "approx." (approximately) denotes an interval of accuracy that a person skilled in the art will understand to still ensure the technical effect of the feature in question. The term typically indicates a deviation from the indicated numerical value of ±20 %, preferably ±15 %, more preferably ±10 %, even more preferably ±5 %, and in particular ±2 %. It is to be understood that the term "comprising" is not limiting. For the purposes of the present invention the term "consisting of" is considered to be a preferred embodiment of the term "comprising".

As used herein, the term "solid content" of a sample refers to the solids in said sample. The solids content SC in the retentate (mainly DIOPAT) was calculated as difference of the evaporation residue (also referred as "dry mass") in retentate minus the evaporation residue of the permeate. The evaporation residues were measured by an infrared drying balance (Mettler Toledo HX204; 110°C; mass constancy for 140 s).

As used herein, the term "aqueous alkaline mixture M" refers to a mixture comprising components (i), (ii), and (iii) as defined herein, which is typically obtained after quenching the Friedel-Crafts reaction mixture to prepare DIOPAT, i.e. component (i), with an aqueous sodium hydroxide solution and removing the organic phase. The pH of the aqueous alkaline mixture M is 10 or more, and is preferably in a range of from 10 to 15, more preferably from 12 to 14, in particular from 12 to 13.5. The amount of Al-salts will typically be in the range of from 1 to 20 % by weight, preferably from 1 to 10 % by weight based on the total weight of the aqueous alkaline mixture M, and the amount of 2,4-DHBP will typically be in the range of from 0.5 to 5 % by weight, preferably from 0.5 to 2 % by weight based on the total weight of the aqueous alkaline mixture M. On the other hand, the amount of DIOPAT will typically be in the range of from 6 to 20 % by weight, preferably from 7 to 14 % by weight, based on the total weight of the aqueous alkaline mixture M.

As used herein "2,4-bis-(2,4-dihydroxyphenyl)-6-(4-methoxyphenyl)-1,3,5-triazine" (DIOPAT) is the compound of interest in the process of the present invention, as it is the precursor for the preparation of Tinosorb^{®} S, as explained above. 2,4-dihydroxybenzophenone is a by-product of its preparation according to one possible synthesis route. As used herein, the term "aluminum salts" (Al-salts) refers to aluminum salts including aluminum trichloride and/or aluminum hydroxide as well as sodium aluminum oxide (NaAlO₂) or mixtures thereof. These aluminum salts are obtained in the preparation of DIOPAT as aluminum trichloride is required for the Friedel-Crafts reaction.

It is to be understood that the aqueous alkaline mixture M may also comprise further components, e.g. sodium chloride, as a result of the reaction of aluminum trichloride with sodium hydroxide upon quenching, as well as sodium hydroxide. In addition, sodium aluminum oxide (NaAlO₂) may be formed upon quenching and therefore be present in the aqueous alkaline mixture M. Furthermore, residual amounts of the starting materials DICAT and resorcinol may be present if the conversion to DIOPAT was incomplete or an excess of DICAT or resorcinol was used.

As used herein, the term "acidified mixture M" refers to a mixture comprising components (i), (ii), and (iii) as defined herein, which is obtained from the aqueous alkaline mixture M after acidification. The acidified mixture M additionally comprises certain amounts of a salt, preferably sodium chloride, from the neutralization reaction. Preferred amounts of the salt obtained due to the neutralization reaction are in the range of from 5 % to 15 % by weight, based on the total weight of the acidified mixture M. Furthermore, instead of a base, such as sodium hydroxide, an acid will be present, preferably hydrogen chloride. The pH of the acidified mixture M is preferably < 1.

As used herein, the term "acidifying" refers to the addition of an acid. Preferred acids include strong inorganic acids, such as sulfuric acid or hydrochloric acid. Preferably, "acidifying" in step i.a) of the process is performed with hydrochloric acid, in particular an aqueous hydrogen chloride solution. Preferred concentrations of the hydrogen chloride solution are in the range of from 20 to 37 %, preferably in the range of from 36 to 37 %. As a result of the acidifying step i.a), sodium chloride may be formed by reaction of sodium hydroxide with hydrogen chloride. As used herein, the term "pH < 1" refers to a pH of below 1.

After the separation step ii.a) of the process by nanofiltration, DIOPAT is obtained in the form of an alkaline aqueous solution S as the retentate. In this connection, the term "aqueous solution S" refers to the solution obtained in step ii.a) comprising DIOPAT. When obtained in step ii.a), the aqueous solution S typically has a pH value of 7 or more or of up to 14, preferably from 7 to 14, more preferably from 8 to 14, in particular from 9 to 13.5, and is therefore referred to as "alkaline aqueous solution S". It is to be understood that the alkaline aqueous solution S may not only comprise DIOPAT, but also residual amounts of Al-salts and 2,4-DHBP, and optionally other impurities as mentioned above. However, the residual amounts of aluminum will typically be below 1 % by weight, preferably below 0.5 % by weight or even below 0.2 % by weight based on the total weight of the alkaline aqueous solution S, and the residual amounts of 2,4-DHBP will typically be below 2 % by weight, preferably below 0.8 % by weight or even below 0.7 % by weight based on the total weight of the alkaline solution, while the amount of DIOPAT will typically be at least 4 % by weight, preferably at least 5 % by weight, or at least 6 % by weight, or at least 7 % by weight, or at least 8 % by weight of the alkaline aqueous solution S. In step ii.b) of said process, the pH of the aqueous solution S is then modified to pH of 9.5 or lower in order to precipitate the DIOPAT. As used herein "modifying the pH to a pH of 9.5 or lower" is performed with an acid. Preferred acids include strong inorganic acids, such as sulfuric acid or hydrochloric acid. Preferably, "modifying the pH to a pH of 9.5 or lower" in step ii.b) of the process is performed with hydrochloric acid, in particular an aqueous hydrogen chloride solution. Preferred concentrations of the hydrogen chloride solution are in the range of from 20 to 37 %, preferably in the range of from 36 to 37 %. Preferably, the pH is modified to a pH of 8 or lower, preferably to a value in the range of from 6 to 8. As a result of the pH modifying step ii.b), additional sodium chloride may be formed by reaction of sodium hydroxide with hydrogen chloride. Thus, after step ii.b), the aqueous solution S preferably comprises an additional amount of sodium chloride.

As used herein, the term "precipitating" refers to solids formation of a compound.

As used herein, the term "diafiltration" (DF) is a subtype of crossflow filtration and refers to a process, wherein a suspension comprising a precipitated compound is separated from dissolved components, which are permeable through a membrane in view of their size. The suspension, which does not pass the membrane is referred to as the "retentate", and the solution comprising the dissolved components, which passes the membrane is referred to as the "permeate". During the diafiltration, the suspension is preferably continuously pumped from the feed vessel to the membrane and from their back to the feed vessel. The diafiltration can be performed as a continuous process, wherein additional solvent is continuously added to the retentate, and the permeate is continuously removed. This results in washing of the precipitated compound in the retentate. Preferably, the diafiltration step i.c) includes washing with water with a washing factor of 3 to 6, preferably 4 to 5, more preferably 4.5, wherein the term "washing factor", also known as "diafiltration factor", refers to the amount of water, also called diafiltration solvent, relative to the suspension as used in step i.c). In this connection, WO2020016366 discloses a suitable process.

As used herein, the expression that "the pH increases from < 1 to at most 3" in the context of step i.c) of the process as below-outlined means that the pH may increase during the diafiltration step due to removal of HCl or other acids with additional solvent, in the present case preferably water. The amount of additional solvent will be selected accordingly. For example, a washing factor of 4.5 results in a pH in the range of from 2 to 3, preferably 2.3 to 2.8. Due to the acidic pH value, dissolution of the precipitated DIOPAT and thereby a decrease of the amount of isolated DIOPAT can be avoided. Of course, it is to be understood that the pH does not necessarily increase to a value as indicated above. The pH may also increase less significantly, if less diafiltration solvent is used. Further, as additional solvent is added in a continuous process over time, the pH value will only increase slowly with increasing amount of diafiltration solvent.

As used herein, the expression that "the temperature remains in the range of from 80°C to 95°C" in the context of step i.c) of the process of the invention means that the temperature of mixture subjected to the diafiltration step, in particular the temperature of the retentate is kept at a temperature of from 80°C to 95°C, e.g., by using a heating device or by adding pre-heated washing water. It is important that the temperature remains in this high range, in order to keep 2,4-dihydroxybenzophenone in solution, so that this byproduct can additionally be removed by the diafiltration step. It is to be understood that the temperature may vary within the range of 80°C to 95°C, when step i.c) of the process is performed. Preferably, the temperature is at the beginning of the diafiltration of step i.c) in a range of from 85°C to 95°C, and then the temperature decreases to a range of from 80°C to 90°C over time, e.g. due to the dilution with additional solvent provided at a temperature in the range of from 80°C to 90°C. Thus, the temperature may be in a range of from 80°C to 90°C, when the diafiltration step has been carried out under continuous addition of additional solvent.

As used herein, the term "concentration factor" refers to the ratio of the starting volume of the suspension comprising the precipitated compound and the dissolved components to the final volume of the suspension comprising the precipitated compound (retentate), after the dissolved components and parts of the solvent (permeate) have been removed during the diafiltration process. It is to be understood that the concentration factor of the additional solvent, which may be continuously added to the retentate, does not affect the concentration factor, as the same amount of additional solvent, which is continuously added to the retentate, will be continuously removed from the system by removing the permeate.

As used herein, "nanofiltration" is performed with a nanofiltration membrane having a pore size of 2 nm or lower. Such nanofiltration membrane are also referred to in the art as having a standard NaCl retention of 70 %. As nanofiltration is performed with an alkaline mixture M according to the present invention, the nanofiltration membrane is preferably a polymer-based nanofiltration membrane, in particular a polyethersulfone membrane filter available, e.g., from Nitto Denko, which is stable in strong alkaline solutions even at elevated temperatures.

In the nanofiltration step ii.a), the DIOPAT is retained from the membrane and therefore obtained in the retentate of the nanofiltration in view of the higher molecular size and high anionic charge. In the filtration step ii.c), the DIOPAT is retained from the membrane and therefore obtained in the retentate of the filtration in view of its precipitated state. As used herein, the term "retentate" refers to the solution or suspension comprising the components, which does not pass the membrane, while the term "permeate" refers to the solution, which passes the membrane and comprises those components, for which the membrane is permeable, i.e. which pass the membrane. In this connection, WO2020089323 discloses a suitable process.

As used herein, the term "suspension" denotes a heterogeneous mixture comprising a solvent and a precipitate. For the diafiltration process, the particles of the precipitate must be larger than the pore size of the membrane.

As used herein, the term "dissolution" or "solution" denotes a homogeneous mixture comprising a solvent and dissolved components, for example ions of a salt. In the diafiltration process, the dissolved components must be sufficiently small that they can pass the membrane.

In step b) of the inventive process, solvent is evaporated from the aqueous suspension S1 by means of an agitated contact dryer. The term "solvent" in this context encompasses any type of solvent present in the aqueous suspension S1, in particular also water.

As used herein, the terms "microfiltration" and "ultrafiltration" both refer to a process, wherein a suspension comprising a precipitated compound is separated from dissolved components, which are permeable through a membrane in view of their size. Typically, the pore size of the membrane used for microfiltration and/or ultrafiltration ranges from about 10 nm to 50 µm, preferably from 20 to 3000 nm, and in particular from 30 to 2000 nm. Further preferred pore sizes may be in the range of 100 nm to 50 µm, preferably of 500 nm to 35 µm, and in particular of 1 to 30 µm. Further preferred pore sizes may be in the range of 10 to 200 nm, preferably of 20 to 150 nm, and in particular of 30 to 80 nm.

Preferred embodiments regarding the process of the invention are described hereinafter.

In a preferred embodiment of the present invention, steps a) to c) are conducted by different means, i.e. by means of different apparatuses.

As already indicated above, the present invention relates to a process for isolating 2,4-bis-(2,4-dihydroxyphenyl)-6-(4-methoxyphenyl)-1,3,5-triazine (DIOPAT) from a DIOPAT-containing aqueous mixture N. Preferably, the DIOPAT-containing aqueous mixture N has a pH of 6 to 14, preferably of 7 to 13, more preferably of 8 to 12, in particular of 9 to 11.

As above-mentioned, DIOPAT may be formed via a Friedel-Crafts reaction of DICAT with resorcinol and subsequent quenching on a pre-charged sodium hydroxide solution. The above-outlined subsequent acidification generally provides an aqueous Al-salts-containing phase, whereas DIOPAT is precipitated as a solid, which may be filtered and neutralized to the desired pH. Hence, it may be one option to provide the aqueous mixture N via the afore-mentioned route.

Alternatively, the DIOPAT-containing aqueous mixture N may be provided via a preceding filtration, e.g. step i)/ii) as further described below. Ultrafiltration and nanofiltration may be named in this connection.

In this connection the DIOPAT-containing aqueous mixture N may be provided via the preceding filtration step i) comprising a process for isolating 2,4-bis-(2,4-dihydroxyphenyl)-6-(4-methoxyphenyl)-1,3,5-triazine (DIOPAT) from an aqueous alkaline mixture M comprising
(i) the 2,4-bis-(2,4-dihydroxyphenyl)-6-(4-methoxyphenyl)-1,3,5-triazine;
(ii) 2,4-dihydroxybenzophenone; and
(iii) aluminum salts;

wherein the process comprises the steps of
   i.a) precipitating the 2,4-bis-(2,4-dihydroxyphenyl)-6-(4-methoxyphenyl)-1,3,5-triazine by acidifying the mixture M to a pH < 1;
   i.b) heating the acidified mixture M to a temperature in the range of from 80°C to 95°C;
   i.c) separating the precipitated 2,4-bis-(2,4-dihydroxyphenyl)-6-(4-methoxyphenyl)-1,3,5-triazine from the dissolved 2,4-dihydroxybenzophenone and the dissolved aluminium salts with a ceramic membrane by means of diafiltration with water, whereby the pH increases from < 1 to at most 3 and the temperature remains in the range of from 80°C to 95°C,
wherein the separation step i.c) provides the precipitated 2,4-bis-(2,4-dihydroxyphenyl)-6-(4-methoxyphenyl)-1,3,5-triazine in the form of an aqueous suspension in the retentate, and the dissolved 2,4-dihydroxybenzophenone and the dissolved aluminum salts in the form of an aqueous solution in the permeate as disclosed in WO2020016366, wherein the process preferably comprises the step of neutralizing i.d) the aqueous suspension of the retentate obtained in step i.c) to obtain a pH of 5 to 11, preferably of 6 to 10, more preferably of 6 to 8, in particular about 7. The aqueous suspension of the retentate may also be adjusted to a pH of 6 to 14, preferably of 7 to 13, more preferably of 8 to 12, in particular of 9 to 11.

Further, the DIOPAT-containing aqueous mixture N may be provided via the preceding nanofiltration step ii) comprising process for isolating 2,4-bis-(2,4-dihydroxyphenyl)-6-(4-methoxyphenyl)-1,3,5-triazine (DIOPAT) from an aqueous alkaline mixture M having a pH of 10 or more and comprising
(i) the 2,4-bis-(2,4-dihydroxyphenyl)-6-(4-methoxyphenyl)-1,3,5-triazine;
(ii) 2,4-dihydroxybenzophenone;
(iii) aluminum salts;
wherein the process comprises the steps of
ii.a) separating the 2,4-bis-(2,4-dihydroxyphenyl)-6-(4-methoxyphenyl)-1,3,5-triazine from the aluminum salts and the 2,4-dihydroxybenzophenone by nanofiltration of the alkaline mixture M, wherein the 2,4-bis-(2,4-dihydroxyphenyl)-6-(4-methoxyphenyl)-1,3,5-triazine is obtained in the form of an alkaline aqueous solution S as the retentate;
ii.b) precipitating the 2,4-bis-(2,4-dihydroxyphenyl)-6-(4-methoxyphenyl)-1,3,5-triazine by modifying the pH of the aqueous solution S to a value of 9.5 or lower;
ii.c) separating the precipitated 2,4-bis-(2,4-dihydroxyphenyl)-6-(4-methoxyphenyl)-1,3,5-triazine from the aqueous solution S by filtration, wherein the 2,4-bis-(2,4-dihydroxyphenyl)-6-(4-methoxyphenyl)-1,3,5-triazine is obtained in the form of an aqueous suspension SP as the retentate;
wherein the nanofiltration step ii.a) is performed at a pressure of from 10 to 40 bar and at a temperature in the range of from 20 to 60°C as disclosed in WO2020089323. Preferably, said process provides the DIOPAT-containing aqueous mixture N having a pH of 6 to 14, preferably of 7 to 13, more preferably of 8 to 12, in particular of 9 to 11.

Preferably, the aqueous mixture N from step a) has a solid content of 0 to less than 10 wt.-%, preferably of 1 to 8 wt. %, in particular of 1 to 6 wt.-%, based on the total weight of the aqueous mixture N. It is particularly preferred that said aqueous mixture N from step a) is provided by a preceding filtration step, preferably by a preceding ultrafiltration step or nanofiltration step. In a particular embodiment, said aqueous mixture N from step a) is provided by a preceding filtration step i) as above described. In another particular embodiment, said aqueous mixture N from step a) is provided by a preceding nanofiltration step ii) as above described.

In a preferred embodiment, the aqueous suspension S1 has a solid content of 4 to 28 wt.-%, preferably of 5 to 26 wt.-%, more preferably of 6 to 25 wt.-%, in particular of more than 6 to 20 wt.-% or of more than 6 to 17 wt.-%, based on the total weight of the aqueous suspension S1. In this connection, the solid content of the aqueous suspension S1 preferably comprises 90 to 99.9 wt.-%, more preferably 95 to 99.7 wt.-%, and in particular 97 to 99.5 wt.-%, of DIOPAT, based on the total weight of the solid content.

Preferably, when comparing the solid content in wt.-% of the aqueous mixture N to the solid content in wt.-% of the aqueous suspension S1 the concentration in step a) provides an increase of the solid content by a factor of 1.2 to 5, more preferably of 1.5 to 4.5, even more preferably of 1.8 to 4.0, and in particular of 2.0 to 3.5.

In a preferred embodiment, the concentrating in step a) is conducted by means of filtration, preferably dynamic crossflow filtration and/or microfiltration.

In another preferred embodiment, the concentrating in step a) is conducted by means of dynamic crossflow filtration, and preferably by microfiltration, wherein the aqueous suspension S1 is the retentate, preferably wherein the discharge flow in step a) is controlled by the concentration of solids, by the density, by the viscosity, and/or by the ratio of permeate to retentate.

In a preferred embodiment, the aqueous mixture N is concentrated in step a) by means of dynamic crossflow filtration, and preferably by microfiltration, wherein the membrane has a pore size from 0.01 to 50 µm, preferably from 0.01 to 5.0 µm, more preferably from 0.1 to 1.5 µm. It is particularly preferred that the membrane has a pore size from 0.01 to 4.0 µm, preferably from 0.05 to 2.0 µm, more preferably from 0.1 to 0.5 µm.

In a preferred embodiment, the aqueous mixture N is concentrated in step a) by means of dynamic crossflow filtration, and preferably by microfiltration, comprising a membrane, wherein the membrane is selected from the group consisting of polymeric membranes, preferably made of polyvinylidene fluoride (PVDF), polysulfone (PSU), polyethersulfone (PES), polyphenylene sulfone (PPSU), polytetrafluoroethylene (PTFE), regenerated cellulose (RC), polyethylen (PE), polypropylen (PP), and mixtures thereof; ceramic materials, preferably aluminum oxide; and metal sieves, preferably made of stainless steel, preferably wherein the membrane material is a polymeric membrane made of polyvinylidene fluoride (PVDF), polyethersulfone (PES), or polytetrafluoroethylene (PTFE), in particular made of polyvinylidene fluoride (PVDF).

Preferably, the membrane has a thermal stability in the range of 40 to 300°C, more preferably of 50 to 200°C, even more preferably of 60 to 150°C, and in particular of 80 to 120°C. In this connection the thermal stability of a polymer membrane is preferably in the range of 40 to 150°C, more preferably of 50 to 130°C, even more preferably of 65 to 110°C, and in particular of 80 to 100°C.

Preferably, the transmembrane pressure (TMP; determined as difference of the pressure of the retentate and the pressure of the permeate) in step a) is in the range of 0.2 to 5 bar, more preferably in the range of 0.3 to 3 bar, even more preferably in the range of 0.4 to 2 bar, and in particular in the range of 0.5 bar to 1 bar.

The average surface velocity is preferably in the range of 1 to 20 m/s, more preferably of 2 to 16 m/s, and in particular of 3 to 10 m/s.

The concentrating in step a) is preferably conducted by means of a single filtration stage or by a series of consecutive filtration stages. In a preferred embodiment, the concentrating in step a) is conducted by 1 to 50 stages, more preferably by 8 to 30 stages, even more preferred by 9 to 21 stages.

In another preferred embodiment, concentrating in step a) is conducted by means of an agitated contact dryer, preferably a thin film evaporator. Any known in the art thin film evaporators may be used. Preferably, the average wall temperature in step b) is from 70 to 250°C, preferably from 90 to 180°C, in particular from 100 to 160°C.

It is to be understood that in the event that concentrating in step a) is performed via an agitated contact dryer, the obtained aqueous suspension S1 is transferred into an additional agitated contact dryer to preform evaporating in step b), preferably wherein these contact dryers are connected in series. The additional contact dryer may be same or different in type.

In a preferred embodiment, evaporating in step b) provides a DIOPAT-containing aqueous suspension S2 having a solid content of 10 to 90 wt.-%, preferably of 20 to 85 wt.-%, more preferably of 30 to 85 wt.-%, in particular of 40 to 80 wt.-%, based on the total weight of the aqueous suspension S2. Particularly preferred is that the evaporating in step b) provides a DIOPAT-containing aqueous suspension S2 having a solid content of 20 to 90 wt.-%, preferably of 40 to 85 wt.-%, more preferably of 60 to 85 wt.-%, in particular of 65 to 80 wt.-%, based on the total weight of the aqueous suspension S2.

Preferably, when comparing the solid content in wt.-% of the aqueous suspension S1 to the solid content in wt.-% of the aqueous suspension S2 the evaporating in step b) provides an increase of the solid content by a factor of 1.5 to 20.0, more preferably of 2.0 to 15.0, even more preferably of 2.5 to 10.0, and in particular of 3.0 to 5.5.

Preferably, the average wall temperature in step b) is from 70 to 250°C, preferably from 90 to 180°C, in particular from 100 to 160°C.

In a preferred embodiment, the agitated contact dryer in step b) is a thin film evaporator. Any known in the art thin film evaporators may be used.

Preferably, the heat transfer coefficients average is in step b) in the range of 100 to 1000 W/m²*K, more preferably of 200 to 800 W/m²*K, and in particular of 400 to 700 W/m²*K. Preferably, step b) is performed under atmospheric conditions.

In the event that a thin film evaporator is used in step b), the rotation is preferably in the range of 200 to 1000 rpm, more preferably of 300 to 900 rpm, even more preferably of 400 to 800 rpm, and in particular of 500 to 700 rpm.

Preferably, the DIOPAT in the DIOPAT-containing suspension S2 in step b) has a particle size of 0.5 to 20 mm, more preferably of 0.8 to 15 mm, and in particular of 1 to 10 mm.

In a preferred embodiment, the DIOPAT in step c) is dried by means of an agitated contact dryer, more preferably a slow rotating linear dryer.

Preferably, the DIOPAT in step c) is dried by means of a disc dryer, more preferably wherein the jacket is heated but the discs are not heated.

Preferably, the temperature in step c) is from 120 to 200°C, more preferably from 130 to 180°C, in particular from 140 to 170°C.

Preferably, the 2,4-bis-(2,4-dihydroxyphenyl)-6-(4-methoxyphenyl)-1,3,5-triazine is obtained in a solid content of more than 93 wt.-%, more preferably of more than 95 wt.-%, and in particular of more than 97 wt.-%, based on the total weight of the 2,4-bis-(2,4-dihydroxyphenyl)-6-(4-methoxyphenyl)-1,3,5-triazine.

In a preferred embodiment, the process is a continuous process, i.e. that the apparatus from steps a) to c) are connected in series.

In this connection, the exhaust gas line preferably comprises a filter in order to avoid carryover of potential dust.

In a preferred embodiment, the overall heat transfer of steps b) and c) is in the range of from 300 to 1600 kW, preferably from 400 to 1300 kW, and in particular form 450 to 800 kW.

Preferably, the inventive process provides DIOPAT having a bulk density, preferably determined by DIN ISO 697 / EN ISO 60, particularly by DIN ISO 697, even more particularly DIN ISO 697:1984, of more than 0.4 g/mL, more preferably of more than 0.5 g/mL, even more preferably of more than 0.6 g/mL and in particular of more than 0.7 g/mL. It is also preferred that the inventive process provides DIOPAT having a bulk density of 0.4 to 1.2 g/mL, more preferably of 0.6 to 1.1 g/mL, even more preferably of more than 0.7 to 1.0 g/mL and in particular of more than 0.8 to 0.9 g/mL.

In a second aspect, the present invention relates to 2,4-bis-(2,4-dihydroxyphenyl)-6-(4-methoxyphenyl)-1,3,5-triazine (DIOPAT) having a bulk density determined by DIN ISO 697, in particular DIN ISO 697:1984, of 0.40 to 1.20 g/mL. Preferably the bulk density determined by DIN ISO 697, in particular DIN ISO 697:1984, of 2,4-bis-(2,4-dihydroxyphenyl)-6-(4-methoxyphenyl)-1,3,5-triazine is of 0.50 to 1.10, more preferably of 0.6 to 1.05 g/mL, even more preferably of more than 0.7 to 1.0 g/mL, and in particular of more than 0.8 to 0.9 g/mL. The 2,4-bis-(2,4-dihydroxyphenyl)-6-(4-methoxyphenyl)-1,3,5-triazine (DIOPAT) of the second aspect of the invention is obtained by the process according to the invention.

### Examples

### Example 1: Microfiltration at 91°C

A neutralized DIOPAT-containing aqueous mixture was produced according to Example UF1 of WO2020016366 having a solid content of about 5.9 wt.-%. The neutralized suspension was further treated as follows.

Microfiltration experiments were conducted using microfiltration membrane MV020 T provided by Microdyn-Nadir.

| supplier | name | Membrane material | Backing material | Pore size [µm] | Maximum temperature [°C] | pH value |
|---|---|---|---|---|---|---|
| Microdyn-Nadir | MV020 T | PVDF | Polyester | 0.2 | 95 | 1-11 |

The fresh feed was provided in the feed drum B1 and pumped by the pump P1 level controlled in the feed vessel B2 (see Figure 1). The feed vessel B2 was pressurized with pressurized air to provide a constant transmembrane pressure (TMP) in the Dynotest filter F1. To get a well-defined discharge of retentate (also called concentrate) even at low retentate flux, the peristaltic discharge system consisting of the pinch valve X1, the pressurized air buffer B3 and the clocked controlled valves H1, H2 and H3 were used. The vessels for the discharged concentrate B5, produced permeate B4 and feed material B1 were continuously weighted. The content of the B2 was recorded by the level in the B2 and a calibration curve.

The apparatus was used in a continuous operation mode. As feed material neutralized Diopat Wet, evaporation residue 12 wt.-% was used. The feed material was produced from the press cake of the filter press by mixing with water, homogenization and neutralization with NaOH. The material was diluted in the drum B1 with demineralized water to a evaporation residue of 3.1 wt.-%. Dry mass (DM) is the total (soluble and insoluble) dry matter in the sample as measured by an infrared drying balance (Mettler Toledo HX204; 110°C; mass constancy for 140 s).

First the feed vessel B2 was filled with the diluted Diopat suspension, stirred and tempered to 91°C. The B2 was pressurized to 1 bar. The rotor of the Dynotest filter F1 was started with an output frequency of the VFD drive of 35 Hz. The average surface velocity was 5.7 m/s.

As soon as produced permeate was collected in the drum B4, the peristaltic discharge system was switched on and concentrate was sent to the drum B5. Using a control loop the pinch valve was periodically actuated after a given waiting time. By variation of the waiting time a constant concentrate mass flow could be established. To get a fixed concentration factor the waiting time was controlled by the ratio of permeate to retentate, (P/R) of the permeate mass flow and the concentrate mass flow-. The P/R ratio was set to 10. The apparatus was operated for two hours. The retentate mass flow was 0.27 kg/h. The permeate mass flow was 2.58 kg/h. Samples of retentate and permeate were taken and analyzed on evaporation residue as described above. The solids content SC in the retentate (mainly DIOPAT) was calculated as difference of dry mass in retentate minus dry mass in permeate. The evaporation residue of the permeate was 0.5 wt.-%. The evaporation residue of the retentate was 25.0 wt.-%. The solids content of the retentate (mainly DIOPAT) was therefore 24.5 wt.-%.

### Example 2: Microfiltration at 46 °C

The apparatus was operated as described in example 1. The feed material was diluted in the drum B1 with demineralized water to an evaporation residue of 1.1 wt.-%.

The temperature in vessel B2 was 46°C. The P/R ratio was set to 14. The apparatus was operated for one hour. The retentate mass flow was 0.31 kg/h. The permeate mass flow was 4.31 kg/h. The evaporation residue of the permeate was 0.2 wt.-%. The evaporation residue of the retentate was 12.1 wt.-%. The solids content of the retentate (mainly DIOPAT) was therefore 11.9 wt.-%.

### Example 3: Drying

A DIOPAT-containing suspension having a solid content of 11.2 wt.-%, was subjected to a thin film evaporator having a heat exchange area of 0.025 m² under the conditions as set out Table 1. The DIOPAT-containing suspension is accessible according to Example 1b of EP0775698, followed by hydrolysis under alkaline conditions, phase separation and a subsequent acidic precipitation. The obtained DIOPAT can then be suspended with the respective amount of water.

**Table 1: Experimental parameters**

| Experiment | Duration feed [min] | Feed rate wet [g/h] | Temperature wall [°C] | Pressure [mbar] | Moisture product [wt.-%] |
|---|---|---|---|---|---|
| T4 | - | - | 200 | atm. | - |

| Remarks: T4 stop of experiment due to plant problem | | | | | |
|---|---|---|---|---|---|
| T5 | 18 | 500 | 200 | atm. | 2.5 |

| Remarks: Stop of experiments due to empty feed container | | | | | |
|---|---|---|---|---|---|
| T6 | 42 | 772 | 200 | atm. | 2.3 |
| T7 | 20 | 1009 | 200 | atm. | 6.9 |
| T8 | 22 | 912 | 200 | atm. | 12.1 |

| Remarks: T8 Dryer revolutions increased compared to all other experiments | | | | | |
|---|---|---|---|---|---|
| T9 | 22 | 910 | 180 | atm. | 3.9 |
| T10 | 31 | 631 | 180 | atm. | 11.0 |
| T11 | 30 | 676 | 180 | atm. | 13.1 |
| T12 | 20 | 457 | 180 | atm. | 3.7 |
| T13 | 20 | 872 | 180 | atm. | 6.5 |
| T14 | 16 | 1080 | 180 | atm. | 3.8 |
| T15 | 32 | 722 | 155 | atm. | 8.6 |
| T16 | 37 | 456 | 155 | atm. | 0.9 |
| T17 | 30 | 320 | 155 | atm. | 0.7 |
| T18 | 20 | 949 | 155 | atm. | 4.0 |
| T19 | 20 | 707 | 155 | 150 | 0.8 |
| T20 | 21 | 992 | 155 | 150 | 0.3 |
| T21 | 20 | 1640 | 155 | 150 | 17.0 |

As can be seen from Table 1 above, a residual moisture content below 3 wt.-% will is unlike to achieve when aiming at higher feed rates.

A DIOPAT-containing suspension having a solid content of 12.0 wt.-%, which has been filtered according to Example UF1 of WO202016366, was subjected to a thin film evaporator having a heat exchange area of 0.025 m² under the conditions as set out in Table 2. It is to be understood that the suspension after ultrafiltration had a solid content of 3.8 to 5.0 wt.-%. For the sake of improved comparison, the suspension was concentrated in the very same thin film apparatus to 12 wt.-% before starting the experiment.

**Table 2: Experimental parameters**

| | | | | | |
|---|---|---|---|---|---|
| Experiment | Duration feed [min] | Feed rate wet [g/h] | Temperature wall [°C] | Pressure [mbar] | Moisture product [wt.-%] |
| T22 | Problems with product feed. Ultrafiltration Diopat more viscous at similar moisture | | | | |
| T23 | | | | | |

| Change of feeding system: Thicker pipes, stirred feed container. | | | | | |
|---|---|---|---|---|---|
| T24 | 20 | 433 | 155 | atm. | 13.6 |
| T25 | 18 | 660 | 155 | atm. | 21.1 |

| Remark: Condensation observed at product container, residual moisture possibly elevated compared to exit moisture. | | | | | |
|---|---|---|---|---|---|
| T26 | 15 | 1013 | 155 | atm. | 25 |
| T27 | 47 | 984 | 155 | atm. | 16.6 |

As can be seen from Table 2 above, it is even more unlikely to obtain a residual moisture content below 3 wt.-% for a DIOPAT-containing suspension with a reduced Al-salt content when aiming at higher feed rates.

A second dryer was connected in series.

A DIOPAT-containing suspension having a solid content of 11.2 wt.-%, which is accessible according to Example 1b of EP0775698 (wherein the product obtained in Example 1b was suspended with the respective amount of water), was subjected to a thin film evaporator having a heat exchange area of 0.025 m² under the conditions as set out in the Table 3.

**Table3: Experimental parameters**

| Experiment | Feed rate [kg/h] | Heating temperature [°C] | rotation [rpm] | Moisture product [wt.-%] |
|---|---|---|---|---|
| P29 | 65 | 155 | 500 | 36.7 |
| P30 | 68 | 155 | 700 | 32.4 - 38 |
| P31 | 71 | 155 | 700 | 33.4 - 34.5 |

In the next examples, a linear dryer was added. A total of 17h of combined operation was established at the parameters provided in Table 4. The residual moisture was at 0.1-0.6 wt.-%, throughout the combined operation.

**Table 4 Experimental parameters**

| Experiment | Feed rate [kg/h] | Heating temperature [°C] | rotation 1^{st} [rpm] | rotation 2^{nd} [rpm] |
|---|---|---|---|---|
| P32 | 69 | 155 | 700 | 30 |
| P33 | 68 | 155 | 700 | 30 |

After the thin film evaporator, DIOPAT particles having the size of 1 to 8 mm have been obtained. The DIOPAT after the linear dryer had a particle size around 1 mm.

The bulk density was determined according to DIN ISO 697.

The DIOPAT from thin film drying and linear drying had a significantly higher bulk density compared to a commercially available (spray-dried) sample, which have been at around 0.38 g/cm³. After the thin film dryer, the bulk density of wet DIOPAT is around 0.7 g/cm³ and the dried powder from the linear dryer reaches bulk densities above 0.8 g/m³. The wide particle size distribution and the relatively large particles in the novel drying route allow for a much better arrangement of the particles and more than twice the bulk density compared to spray drying. The spray dried DIOPAT powder is small enough (d90 at around 120 µm) that cohesion forces in the particle come into play, which typically hinder a perfect arrangement of the bulk powder. Details may be derived from Table 5.

**Table 5: DIOPAT measured bulk densities**

| Sample No. | Origin | Weight [g] | Volume [mL] | Density [g/cm³] | Density average (3) [g/cm³] |
|---|---|---|---|---|---|
| | commercial sample | 37.82 | 100.15 | 0.3776 | 0.3776 |
| | | 37.70 | 100.15 | 0.3764 | |
| | | 37.94 | 100.15 | 0.3788 | |
| P31-T1 | Exit thin film dryer | 69.09 | 100.15 | 0.6899 | 0.6878 |
| | | 68.67 | 100.15 | 0.6857 | |
| | | 68.88 | 100.15 | 0.6878 | |
| P32-T1 | Exit thin film dryer | 72.86 | 100.15 | 0.7275 | 0.7351 |
| | | 74.23 | 100.15 | 0.7412 | |
| | | 73.76 | 100.15 | 0.7265 | |
| P32-T2 | Exit linear dryer | 84.79 | 100.15 | 0.8466 | 0.8531 |
| | | 85.81 | 100.15 | 0.8568 | |
| | | 85.71 | 100.15 | 0.8558 | |
| P33-T2 | Exit linear dryer | 83.06 | 100.15 | 0.8294 | 0.8291 |
| | | 82.17 | 100.15 | 0.8205 | |
| | | 83.87 | 100.15 | 0.8374 | |

### Example 4: Mass and energy balance

The thin film dryer on pilot scale had an effective heat exchange area of 1.5 m². At 155°C wall temperature it could process 70 kg/h of Diopat feed with about 12 wt.-% solids concentration (compare Example 3). Thin film dryers scale up according to the heat exchange area. Hence, using an adapted heat exchange area, the above-outlined process can be scaled-up as necessary in order to provide an energy saving Diopat separation.

## Claims

1. A process for isolating 2,4-bis-(2,4-dihydroxyphenyl)-6-(4-methoxyphenyl)-1,3,5-triazine
(DIOPAT) from a 2,4-bis-(2,4-dihydroxyphenyl)-6-(4-methoxyphenyl)-1,3,5-triazine-containing aqueous mixture N comprising the steps of
a) concentrating the aqueous mixture N to obtain a 2,4-bis-(2,4-dihydroxyphenyl)-6-(4-methoxyphenyl)-1,3,5-triazine-containing aqueous suspension S1 having a solid content of 3 to 30 wt.-%, based on the total weight of the aqueous suspension S1;
b) evaporating solvent from the aqueous suspension S1 by means of an agitated contact dryer; and
c) drying the 2,4-bis-(2,4-dihydroxyphenyl)-6-(4-methoxyphenyl)-1,3,5-triazine to obtain a solid content of more than 90 wt.-%, based on the total weight of the 2,4-bis-(2,4-dihydroxyphenyl)-6-(4-methoxyphenyl)-1,3,5-triazine;
wherein the term "solvent" in this context encompasses any type of solvent present in the aqueous suspension S1, including water.

2. The process according to claim 1, wherein the aqueous suspension S1 has a solid content of 4 to 28 wt.-%, preferably of 5 to 26 wt.-%, more preferably of 6 to 25 wt.-%, in particular of more than 6 to 20 wt.-% or of more than 6 to 17 wt.-%, based on the total weight of the aqueous suspension S1.

3. The process according to claim 1 or 2, wherein the aqueous mixture N has a pH 6 to 14, preferably of 7 to 13, more preferably of 8 to 12, in particular of 9 to 11.

4. The process according to any one of claims 1 to 3, wherein concentrating in step a) is conducted by means of filtration.

5. The process according to any one of claims 1 to 3, wherein concentrating in step a) is conducted by means of dynamic crossflow filtration, wherein the aqueous suspension S1 is the retentate, preferably wherein the discharge flow in step a) is controlled by the concentration of solids, by the density, by the viscosity, and/or by the ratio of permeate to retentate.

6. The process according to any one of claims 1 to 5, wherein the aqueous mixture N is concentrated in step a) by means of dynamic crossflow filtration comprising a membrane, wherein the membrane has a pore size from 0.01 to 50 µm, preferably from 0.01 to 5.0 µm, more preferably from 0.1 to 1.5 µm and/or
the membrane material is selected from the group consisting of polymeric membranes, preferably made of polyvinylidene fluoride (PVDF), polysulfone (PSU), polyethersulfone (PES), polyphenylene sulfone (PPSU), polytetrafluoroethylene (PTFE), regenerated cellulose (RC), polyethylen (PE), polypropylen (PP), and mixtures thereof; ceramic materials, preferably aluminum oxide; and metal sieves, preferably made of stainless steel, preferably wherein the membrane material is a polymeric membrane made of polyvinylidene fluoride (PVDF).

7. The process according to any one of claims 1 to 3, wherein concentrating in step a) is conducted by means of an agitated contact dryer, preferably a thin film evaporator.

8. The process according to any one of claims 1 to 7, wherein the agitated contact dryer in step b) is a thin film evaporator.

9. The process according to any one of claims 1 to 8, wherein evaporating in step b) provides a 2,4-bis-(2,4-dihydroxyphenyl)-6-(4-methoxyphenyl)-1,3,5-triazine-containing aqueous suspension S2 having a solid content of 10 to 90 wt.-%, preferably of 20 to 85 wt.- %, more preferably of 30 to 85 wt.-%, in particular of 40 to 80 wt.-%, based on the total weight of the aqueous suspension S2.

10. The process according to any one of claims 1 to 9, wherein the average wall temperature in step b) is from 70 to 250 °C, preferably from 90 to 180 °C, in particular from 100 to 160 °C.

11. The process according to any one of claims 1 to 10, wherein the 2,4-bis-(2,4-dihydroxyphenyl)-6-(4-methoxyphenyl)-1,3,5-triazine in step c) is dried by means of an agitated contact dryer, preferably a slow rotating linear dryer.

12. The process according to any one of claims 1 to 11, wherein the 2,4-bis-(2,4-dihydroxyphenyl)-6-(4-methoxyphenyl)-1,3,5-triazine in step c) is dried by means of a disc dryer, preferably wherein the jacket is heated but the discs are not heated.

13. The process according to any one of claims 1 to 12, wherein the temperature in step c) is from 120 to 200 °C, preferably from 130 to 180 °C, in particular from 140 to 170 °C.

14. The process according to any one of claims 1 to 13, wherein the process is a continuous process.

15. The process according to any one of claims 1 to 14, wherein the aqueous mixture N from step a) has a solid content of 0 to less than 10 wt.-%, preferably of 1 to 8 wt.-%, in particular of 1 to 6 wt.-%, based on the total weight of the aqueous mixture N and/or is provided by a preceding filtration step, preferably by a preceding ultrafiltration step or nanofiltration step.

16. 2,4-bis-(2,4-dihydroxyphenyl)-6-(4-methoxyphenyl)-1,3,5-triazine (DIOPAT) obtained by the process of one of claims 1 to 15 having a bulk density determined by DIN ISO 697:1984 of 0.40 to 1.20 g/mL.

## Patentansprüche

1. Verfahren zur Isolierung von 2,4-Bis-(2,4-dihydroxyphenyl)-6-(4-methoxyphenyl)-1,3,5-triazin (DIOPAT) aus einer 2,4-Bis-(2,4-dihydroxyphenyl)-6-(4-methoxyphenyl)-1,3,5-triazin enthaltenden wässrigen Mischung N, umfassend die Schritte:
a) Konzentrieren der wässrigen Mischung N, um eine 2,4-Bis-(2,4-dihydroxyphenyl)-6-(4-methoxyphenyl)-1,3,5-triazin enthaltende wässrige Suspension S1 mit einem Feststoffgehalt von 3 bis 30 Gew.-%, bezogen auf das Gesamtgewicht der wässrigen Suspension S1, zu erhalten;
b) Verdampfen von Lösungsmittel aus der wässrigen Suspension S1 mithilfe eines gerührten Kontakttrockners; und
c) Trocknen des 2,4-Bis-(2,4-dihydroxyphenyl)-6-(4-methoxyphenyl)-1,3,5-triazins, um einen Feststoffgehalt von mehr als 90 Gew.-%, bezogen auf das Gesamtgewicht des 2,4-Bis-(2,4-dihydroxyphenyl)-6-(4-methoxyphenyl)-1,3,5-triazins, zu erhalten;
wobei der Begriff "Lösungsmittel" in diesem Zusammenhang eine beliebige Art von Lösungsmittel umfasst, das in der wässrigen Suspension S1 vorhanden ist, einschließlich Wasser.

2. Verfahren nach Anspruch 1, wobei die wässrige Suspension S1 einen Feststoffgehalt von 4 bis 28 Gew.-%, bevorzugt von 5 bis 26 Gew.-%, bevorzugter von 6 bis 25 Gew.-%, insbesondere von mehr als 6 bis 20 Gew.-% oder mehr als 6 bis 17 Gew.-%, bezogen auf das Gesamtgewicht der wässrigen Zusammensetzung S1, aufweist.

3. Verfahren nach Anspruch 1 oder 2, wobei die wässrige Mischung N einen pH-Wert von 6 bis 14, bevorzugt 7 bis 13, bevorzugter 8 bis 12, insbesondere 9 bis 11 aufweist.

4. Verfahren nach einem der Ansprüche 1 bis 3, wobei Konzentrieren in Schritt a) mithilfe von Filtration durchgeführt wird.

5. Verfahren nach einem der Ansprüche 1 bis 3, wobei Konzentrieren in Schritt a) mithilfe von dynamischer Querstromfiltration durchgeführt wird, wobei die wässrige Suspension S1 das Retentat ist, wobei der Austragsstrom in Schritt a) vorzugsweise durch die Konzentration von Feststoffen, durch die Dichte, durch die Viskosität und/oder durch das Verhältnis von Permeat zu Retentat gesteuert wird.

6. Verfahren nach einem der Ansprüche 1 bis 5, wobei die wässrige Mischung N in Schritt a) mithilfe von dynamischer Querstromfiltration, umfassend eine Membran, konzentriert wird, wobei die Membran eine Porengröße von 0,01 bis 50 µm, bevorzugt von 0,01 bis 5,0 µm, besonders bevorzugt von 0,1 bis 1,5 µm aufweist und/oder das Membranmaterial ausgewählt ist aus der Gruppe bestehend aus polymeren Membranen, vorzugsweise hergestellt aus Polyvinylidenfluorid (PVDF), Polysulfon (PSU), Polyethersulfon (PES), Polyphenylensulfon (PPSU), Polytetrafluorethylen (PTFE), regenerierter Cellulose (RC), Polyethylen (PE), Polypropylen (PP) und Mischungen davon; keramischen Materialien, vorzugsweise Aluminiumoxid; und Metallsieben, die vorzugsweise aus rostfreiem Stahl hergestellt sind, wobei das Membranmaterial vorzugsweise eine Polymermembran ist, die aus Polyvinylidenfluorid (PVDF) hergestellt ist.

7. Verfahren nach einem der Ansprüche 1 bis 3, wobei Konzentrieren in Schritt a) mithilfe eines gerührten Kontakttrockners, vorzugsweise eines Dünnfilmverdampfers, durchgeführt wird.

8. Verfahren nach einem der Ansprüche 1 bis 7, wobei der gerührte Kontakttrockner in Schritt b) ein Dünnfilmverdampfer ist.

9. Verfahren nach einem der Ansprüche 1 bis 8, wobei Verdampfen in Schritt b) eine 2,4-Bis-(2,4-dihydroxyphenyl)-6-(4-methoxyphenyl)-1,3,5-triazin enthaltende wässrige Suspension S2 mit einem Feststoffgehalt von 10 bis 90 Gew.-%, bevorzugt von 20 bis 85 Gew.-%, bevorzugter von 30 bis 85 Gew.-%, insbesondere von 40 bis 80 Gew.-%, bezogen auf das Gesamtgewicht der wässrigen Suspension S2, bereitstellt.

10. Verfahren nach einem der Ansprüche 1 bis 9, wobei die durchschnittliche Wandtemperatur in Schritt b) von 70 bis 250 °C, bevorzugt von 90 bis 180 °C, insbesondere von 100 bis 160 °C beträgt.

11. Verfahren nach einem der Ansprüche 1 bis 10, wobei das 2,4-Bis-(2,4-dihydroxyphenyl)-6-(4-methoxyphenyl)-1,3,5-triazin in Schritt c) mithilfe eines gerührten Kontakttrockners, vorzugsweise eines langsam rotierenden Lineartrockners getrocknet wird.

12. Verfahren nach einem der Ansprüche 1 bis 11, wobei das 2,4-Bis-(2,4-dihydroxyphenyl)-6-(4-methoxyphenyl)-1,3,5-triazin in Schritt c) mittels eines Scheibentrockners getrocknet wird, wobei vorzugsweise der Mantel erwärmt wird, die Scheiben jedoch nicht erwärmt werden.

13. Verfahren nach einem der Ansprüche 1 bis 12, wobei die Temperatur in Schritt c) von 120 °C bis 200 °C, vorzugsweise von 130 °C bis 180 °C, insbesondere von 140 °C bis 170 °C ist.

14. Verfahren nach einem der Ansprüche 1 bis 13, wobei das Verfahren ein kontinuierliches Verfahren ist.

15. Verfahren nach einem der Ansprüche 1 bis 14, wobei die wässrige Mischung N aus Schritt a) einen Feststoffgehalt von 0 bis weniger als 10 Gew.-%, bevorzugt von 1 bis 8 Gew.-%, insbesondere von 1 bis 6 Gew.-%, bezogen auf das Gesamtgewicht der wässrigen Mischung N, aufweist und/oder durch einen vorhergehenden Filtrationsschritt, vorzugsweise durch einen vorhergehenden Ultrafiltrationsschritt oder Nanofiltrationsschritt, bereitgestellt wird.

16. 2,4-Bis-(2,4-dihydroxyphenyl)-6-(4-methoxyphenyl)-1,3,5-triazin
(DIOPAT), erhalten nach dem Verfahren nach einem der Ansprüche 1 bis 15, mit einer Schüttdichte, bestimmt nach DIN ISO 697:1984, von 0,40 bis 1,20 g/ml.

## Revendications

1. Procédé pour isoler la 2,4-bis-(2,4-dihydroxyphényl)-6-(4-méthoxyphényl)-1,3,5-triazine (DIOPAT) à partir d'un mélange aqueux N contenant de la 2,4-bis-(2,4-dihydroxyphényl)-6-(4-méthoxyphényl)-1,3,5-triazine comprenant les étapes de
a) concentration du mélange aqueux N pour obtenir une suspension aqueuse S1 contenant de la 2,4-bis-(2,4-dihydroxyphényl)-6-(4-méthoxyphényl)-1,3,5-triazine ayant une teneur en solides de 3 à 30 % en poids, par rapport au poids total de la suspension aqueuse S1 ;
b) évaporation de solvant de la suspension aqueuse S1 au moyen d'un séchoir agité par contact ; et
c) séchage de la 2,4-bis-(2,4-dihydroxyphényl)-6-(4-méthoxyphényl)-1,3,5-triazine pour obtenir une teneur en solides de plus de 90 % en poids par rapport au poids total de la 2,4-bis-(2,4-dihydroxyphényl)-6-(4-méthoxyphényl)-1,3,5-triazine ;
dans lequel le terme « solvant », dans ce contexte, englobe tout type de solvant présent dans la suspension aqueuse S1, y compris l'eau.

2. Procédé selon la revendication 1, dans lequel la suspension aqueuse S1 possède une teneur en solides de 4 à 28 % en poids, de préférence de 5 à 26 % en poids, plus préférablement de 6 à 25 % en poids, en particulier supérieure à 6 à 20 % en poids ou supérieure à 6 à 17 % en poids, par rapport au poids total de la suspension aqueuse S1.

3. Procédé selon la revendication 1 ou 2, dans lequel le mélange aqueux N possède un pH 6 à 14, de préférence de 7 à 13, plus préférablement de 8 à 12, en particulier de 9 à 11.

4. Procédé selon l'une quelconque des revendications 1 à 3, dans lequel la concentration dans l'étape a) est effectuée au moyen d'une filtration.

5. Procédé selon l'une quelconque des revendications 1 à 3, dans lequel la concentration dans l'étape a) est effectuée au moyen d'une filtration dynamique à écoulement transversal, dans lequel la suspension aqueuse S1 est le rétentat, de préférence dans lequel l'écoulement de décharge dans l'étape a) est régulé par la concentration de solides, par la densité, par la viscosité et/ou par le rapport perméat sur rétentat.

6. Procédé selon l'une quelconque des revendications 1 à 5, dans lequel le mélange aqueux N est concentré dans l'étape a) au moyen d'une filtration dynamique à écoulement transversal comprenant une membrane, dans lequel la membrane a une taille de pores de 0,01 à 50 µm, de préférence de 0,01 à 5,0 µm, plus préférablement de 0,1 à 1,5 µm et/ou
le matériau de membrane est choisi dans le groupe constitué par les membranes polymériques, de préférence composées de poly(fluorure de vinylidène) (PVDF), polysulfone (PSU), polyéthersulfone (PES), polyphénylène sulfone (PPSU), polytétrafluoroéthylène (PTFE), cellulose régénérée (RC), polyéthylène (PE), polypropylène (PP), et leurs mélanges ; les matériaux de céramique, de préférence l'oxyde d'aluminium ; et les tamis métalliques, de préférence composés d'acier inoxydable, de préférence dans lequel le matériau de membrane est une membrane polymérique composée de poly(fluorure de vinylidène) (PVDF).

7. Procédé selon l'une quelconque des revendications 1 à 3, dans lequel la concentration dans l'étape a) est effectuée au moyen d'un séchoir agité par contact, de préférence un évaporateur de film mince.

8. Procédé selon l'une quelconque des revendications 1 à 7, dans lequel le séchoir agité par contact dans l'étape b) est un évaporateur de film mince.

9. Procédé selon l'une quelconque des revendications 1 à 8, dans lequel l'évaporation dans l'étape b) fournit une suspension aqueuse S2 contenant de la 2,4-bis-(2,4-dihydroxyphényl)-6-(4-méthoxyphényl)-1,3,5-triazine ayant une teneur en solides de 10 à 90 % en poids, de préférence de 20 à 85 % en poids, plus préférablement de 30 à 85 % en poids, en particulier de 40 à 80 % en poids, par rapport au poids total de la suspension aqueuse S2.

10. Procédé selon l'une quelconque des revendications 1 à 9, dans lequel la température moyenne de paroi dans l'étape b) est de 70 °C à 250 °C, de préférence de 90 °C à 180 °C, en particulier de 100 °C à 160 °C.

11. Procédé selon l'une quelconque des revendications 1 à 10, dans lequel la 2,4-bis-(2,4-dihydroxyphényl)-6-(4-méthoxyphényl)-1,3,5-triazine dans l'étape c) est séchée au moyen d'un séchoir agité par contact, de préférence un séchoir linéaire à rotation lente.

12. Procédé selon l'une quelconque des revendications 1 à 11, dans lequel la 2,4-bis-(2,4-dihydroxyphényl)-6-(4-méthoxyphényl)-1,3,5-triazine dans l'étape c) est séchée au moyen d'un séchoir à disques, de préférence dans lequel l'enveloppe est chauffée mais les disques ne sont pas chauffés.

13. Procédé selon l'une quelconque des revendications 1 à 12, dans lequel la température dans l'étape c) est de 120 °C à 200 °C, de préférence de 130 °C à 180 °C, en particulier de 140 °C à 170 °C.

14. Procédé selon l'une quelconque des revendications 1 à 13, dans lequel le procédé est un procédé continu.

15. Procédé selon l'une quelconque des revendications 1 à 14, dans lequel le mélange aqueux N de l'étape a) a une teneur en solides de 0 à moins de 10 % en poids, de préférence de 1 à 8 % en poids, en particulier de 1 à 6 % en poids, par rapport au poids total du mélange aqueux N et/ou est fourni par une étape de filtration précédente, de préférence par une étape d'ultrafiltration ou étape de nanofiltration précédente.

16. 2,4-bis-(2,4-dihydroxyphényl)-6-(4-méthoxyphényl)-1,3,5-triazine
(DIOPAT) obtenue par le procédé selon l'une des revendications 1 à 15 ayant une densité apparente déterminée par la norme DIN ISO 697:1984, de 0,40 à 1,20 g/mL.
